# EUROPEAN PATENT APPLICATION

(11) **EP 4 685 736 A1**
(43) Date of publication of application: **28.01.2026**
(21) Application number: 24306245.2
(22) Date of filing: 23.07.2024
(51) Int. Cl.: G06T 7/00, A61B 6/50, G06T 7/30

(54) **MEDICAL IMAGE PROCESSING METHOD, COMPUTER PROGRAM ELEMENT AND IMAGE PROCESSING DEVICE**

(71) Applicant: Koninklijke Philips N.V., 5656 AG Eindhoven (NL)
(72) Inventor: WIEMKER, Rafael, 5656 AG Eindhoven (NL); VLACHOMITROU, Anna Sesilia, 5656 AG Eindhoven (NL); VILLIEN, Marjorie, 5656 AG Eindhoven (NL); PETERS, Jochen, 5656 AG Eindhoven (NL); BONTUS, Claas, 5656 AG Eindhoven (NL)
(74) Representative: Philips Intellectual Property & Standards

(57) **Abstract**

The invention relates to a medical image processing method (1). According to the method, cardiac image data of a patient relating to at least two different cardiac characteristics is received (S1). The cardiac image data is processed (S2) to generate a synoptic representation (2) of the at least two different cardiac characteristics and the synoptic representation (2) is output (S3). The invention further relates to a corresponding computer program element and to a corresponding image processing device.

## Description

### FIELD OF THE INVENTION

The invention relates to medical image processing, in particular to processing of cardiac image data. More particularly, the invention relates to a medical image processing method, to a computer program element and to an image processing device.

### BACKGROUND OF THE INVENTION

For the assessment of the myocardial viability, changes in perfusion between rest and stress scans, as well as late enhancement scans, are an important parameter. Another important parameter is the local magnitude of wall motion or a wall thickness change between different cardiac phases reconstructed from a certain CT scan. Visual appraisal can reveal, e.g., local perfusion defects and local wall motion abnormalities.

Both of the above-mentioned analyses can be used to assess, e.g., myocardial infarction, ischemia and therapeutic viability. However, each finding alone can be inconclusive, hence it is crucial to check for spatial correspondence or lack thereof between the various patterns.

To find the spatial correspondence, four or more image volumes from multiple scans and reconstructions may be simultaneously appraised. However, this is tedious and prone to visual inconclusiveness. Moreover, it is very demanding to estimate a change in perfusion and location or thickness, respectively, at the same time as spatial correspondence of the change patterns by unaided visual appraisal.

### SUMMARY OF THE INVENTION

It is an object of the present invention to provide a medical image processing method that allows for an improved assessment of a myocardium of a patient. It is a further object of the present invention to provide a corresponding computer program element and a corresponding image processing device.

The object of the present invention is solved by the subject-matter of the independent claims, wherein further embodiments are incorporated in the dependent claims.

In an aspect of the present invention, a medical image processing method is provided.

According to the method, cardiac image data of a patient relating to at least two different cardiac characteristics is received. In this context, the patient may be a human, but may also be an animal. The cardiac image data may have been obtained using computed tomography (CT), in particular spectral CT or photon-counting CT. Alternatively, or additionally, the cardiac image data may have been obtained using positron emission tomography (PET) or magnetic resonance imaging (MRI). The cardiac image data relates to at least two different cardiac characteristics. Depending on the characteristics, the cardiac image data may be obtained in a same scan sequence, in different scan sequences, or even in a single scan.

The cardiac image data is processed to generate a synoptic representation of the at least two different cardiac characteristics. In this context, a synoptic representation is a representation that shows the at least two different cardiac characteristics together, such that a combined assessment of the at least two different cardiac characteristics is enabled.

The synoptic representation is then output. Said output may be, e.g., directly to a screen, or via a graphics interface and/or a network to a screen. The output may also be to a memory, such that the synoptic representation can be accessed from the memory for later display and assessment.

The synoptic representation may be viewed by a clinician to assess a myocardium of the patient. In particular, the synoptic representation may be used to assess myocarditis or ischemia, or for cardiac quantification and/or diagnosis. Since at least two different cardiac characteristics are assessed together using the synoptic representation, a spatial correspondence between defect patterns seen in each of the at least two different cardiac characteristics may be identified, leading to an improved assessment of the myocardium of the patient and hence to an improvement of the diagnostic certainty.

According to an embodiment, the cardiac characteristics comprise a perfusion of a myocardium of the patient. In this context, the myocardium may be the entire myocardium of the patient, or a part of the myocardium of the patient, in particular, the part of the myocardium corresponding to the left ventricle of the heart. The perfusion may be determined by the uptake of a contrast agent. In particular, the perfusion may be determined when the patient is at rest or when the patient is at stress, more particularly at induced stress. Further, the perfusion may be measured in an arterial phase, which is also called late systemic-arterial phase or early venous portal phase. Alternatively, the perfusion may be measured in a late enhancement phase, which is also called delayed enhancement phase or delayed phase. Said late enhancement phase is particularly sensitive to tissue with fibrosis. Yet alternatively, the perfusion may be measured in another phase of the contrast enhancement that is deemed useful. Four specific cardiac characteristics for perfusion may be derived from this list: at rest and measured in the arterial phase, at rest and measured in the late enhancement phase, at stress and measured in the arterial phase, and at stress and measured in the late enhancement phase, and other cardiac characteristics can easily be thought of. Additionally, or alternatively, the cardiac characteristics comprise a change in perfusion of the myocardium of the patient. This may be, in particular, a change between a measurement of the patient at rest and another measurement at stress. Additionally, or alternatively, the cardiac characteristics comprise a wall thickness of the myocardium. Said wall thickness may be measured, in particular, at a diastole or at a systole, but measurements at other cardiac phases are also possible. Said measurements at specific cardiac phases may be performed by ECG-gated scans of the beating heart. Additionally, or alternatively, the cardiac characteristics may comprise a change in wall thickness of the myocardium of the patient. This may be, in particular, a change between the wall thickness measured at the diastole and the wall thickness measured at the systole. Additionally, or alternatively, the cardiac characteristics may comprise a magnitude of wall motion of the myocardium of the patient. In particular, the magnitude of wall motion may be the magnitude of the motion of the endo-cardiac wall, the magnitude of the motion of the epi-cardiac wall, or the magnitude of the motion of a layer between the endo-cardiac wall and the epi-cardiac wall. The magnitude of wall motion may be determined, e.g., by the motion of the wall between the diastole and the systole. The magnitude of wall motion is understood as a local magnitude and is indicative of myocardial contractility. In summary, there is a plurality of different cardiac characteristics that may be used to generate the synoptic representation. Using at least two cardiac characteristics that are complementary to one another for the synoptic representation results in a particularly advantageous embodiment of the method. Such cardiac characteristics that are complementary to one another are, in particular, perfusion characteristics on the one hand and wall thickness or wall motion characteristics on the other hand. An appraisal of these cardiac characteristics together leads to an improved assessment of the myocardium.

According to an embodiment, the synoptic representation comprises at least one graphical representation of the myocardium that temporally changes between the at least two different cardiac characteristics. That is, there is at least one graphical representation, and the data presented in this graphical representation changes in time between the at least two different cardiac characteristics. Since there is a given graphical representation, finding spatial correspondences is facilitated and hence the assessment of the myocardium is improved.

According to an embodiment, the temporal change between the at least two different cardiac characteristics is a discrete switch. That is, the data presented in the graphical representation changes suddenly from one cardiac characteristic to another cardiac characteristic. For example, the data presented in the graphical representation changes suddenly from a perfusion characteristic to the magnitude of wall motion. Alternatively, or additionally, the temporal change between the at least two different cardiac characteristics is a smooth blend. That is, the data presented in the graphical representation changes smoothly from one cardiac characteristic to another cardiac characteristic. This smooth change may be achieved by making interpolations, e.g., linear interpolations, between the corresponding values of the cardiac characteristics. Also, if shapes are involved in the graphical representation, interpolations between these shapes, e.g., between three-dimensional meshes, may be performed. For example, the data presented in the graphical representation changes smoothly from a perfusion at rest to a perfusion at stress, or from a wall thickness at a diastole to a wall thickness at a systole. As an example for a combination of a discrete switch and a smooth blend, the data presented in the graphical representation changes suddenly from a change in perfusion to a wall thickness at the diastole, and then changes smoothly from the wall thickness at the diastole to the wall thickness at the systole.

According to an embodiment, the step of processing the cardiac image data comprises receiving user input, and the temporal change between the at least two different cardiac characteristics is controlled by the user input. Said user input may be provided using dedicated hardware such as switches or sliders or using corresponding features on a graphical user interface. As an example, for two different cardiac characteristics, the user input may be provided with a switch for the discrete switch change or with a slider for the smooth blend change. As another example, for a plurality of cardiac characteristics and a discrete switch change, the cardiac characteristics may be provided in a list structure and the user may activate each of the cardiac characteristics separately. As yet another example, for a plurality of cardiac characteristics and a smooth blend change, a plurality of sliders or a two-dimensional input surface may be used to obtain the user input. Since the user can control the temporal change between the at least two different cardiac characteristics, he/she can adapt said temporal change and the displayed data such that an optimal assessment of the myocardium is enabled.

Alternatively, the temporal change may be performed automatically, e.g., by periodically switching or blending between different cardiac characteristics. The frequency of said periodical switching or blending may be adjusted by the user, and the periodical switching may also be paused by the user.

According to an embodiment, the synoptic representation comprises at least one graphical representation of the myocardium with interleaved alternating patches corresponding to the at least two different cardiac characteristics. Hence, the at least two different cardiac characteristics can be seen at the same time, albeit not for the entire area. In particular, the interleaved alternating patches may resemble a checkerboard pattern, which provides a clearly discernible structure. This embodiment may also be combined with the temporal change described above. As an example, for two different cardiac characteristics, a certain patch may be shown with data of the first cardiac characteristic, and a neighboring patch may be shown with data of the second cardiac characteristic. This representation may temporally change with a representation where the certain patch is shown with data of the second cardiac characteristic and the neighboring patch is shown with data of the first cardiac characteristic. Again, said temporal change may be a discrete switch and/or a smooth blend, and may be controlled by user input.

According to an embodiment, the at least one graphical representation comprises at least one out of a 17-segment model view, a two-dimensional projection, a color overlay on a cross-sectional view, and a three-dimensional surface rendering decorated with a texture. The 17-segment model view is a polar projection according to the American heart association (AHA), yielding a bull's eye plot. The 17 segments are also referred to as AHA segments. The two-dimensional projection of the myocardium may be, e.g., a conformal Mercator projection or an area-true Mollweide or Hammer projection. The color overlay on a cross-sectional view may also be referred to as color overlay on slice images. Said slice images may be reformatted along a long or a short ventricle axis and may be masked for the myocardium. In the three-dimensional surface rendering decorated with a texture, a functional map is draped as texture over a three-dimensional surface rendering of the cardiac wall, e.g., as a textured triangle mesh rendering, or a thin-shell volume rendering. The texture corresponds to the data of the cardiac characteristic and may correspond to the two-dimensional projection.

According to an embodiment, the cardiac image data is volumetric image data and the step of processing the cardiac image data comprises performing a cardiac segmentation on the volumetric image data to obtain a myocardial region within the volumetric image data. In particular, the myocardial region may be automatically detected and segmented on each of the image volumes. This automatic segmentation may be based on, e.g., model-based segmentation or other machine learning-based semantic segmentations such as deep convolutional neural networks.

According to an embodiment, the step of processing the cardiac image data comprises receiving further user input, and the myocardial region is refined based on the further user input. Said further user input may be provided, e.g., via a graphical user interface. This manual contour refinement may be performed when the user notices delineation imperfections and leads to better contours and hence an improved assessment of the myocardium.

Alternatively, the cardiac image data is pre-processed data, e.g., data on which the detection and segmentation of the myocardial region has already been performed. In this case, the above-mentioned step does not have to be performed.

According to an embodiment, the step of processing the cardiac image data comprises performing spatial alignment and/or registration. In particular, all image volumes may be mutually spatially aligned and co-registered. As an example, for the wall motion analysis, it is necessary to remove global displacements of the heart between scans, but not changes in size or shape. Therefore, this alignment may be carried out in a rigid, i.e., non-elastic, registration, e.g., by aligning myocardial centroid coordinates. As another example, for the perfusion analysis, it is necessary to establish elastic correspondences, i.e., a deformable registration, which is achieved, e.g., by landmark correspondences such as mesh triangle labels, by AHA-segment-correspondence, or by full-volume voxelwise image-based registration. The spatial alignment and/or registration is not only important for the above-mentioned analyses, but also for generating a consistent view between the different cardiac characteristics, allowing for a good assessment of spatial correspondences.

According to an embodiment, the step of processing the cardiac image data comprises performing a normalization of values of each cardiac characteristic. For said normalization, a reference value may be determined, e.g., by a median, a mean, a maximum, or a minimum, and deviations from said reference value may be expressed in terms of, e.g., a standard deviation, or an interquartile range. In particular, this normalization may be performed on the entire myocardium, the part of the myocardium corresponding to the left ventricle, or the ventricular blood-pool region. As an example, since perfusion is signified by a radio-opacity of the administered contrast agent (yielding Hounsfield values in the computed tomography image data), and concentration levels of the contrast agent may vary between scans, the normalization allows a comparison between the data of the scans.

According to an embodiment, the step of processing the cardiac image data comprises mapping values of each cardiac characteristic to a gray-level scale or a pseudo-color scale. It is to be noted that for different cardiac characteristics, the same scale may be used or different scales may be used. When different scales are used, e.g., a red-blue scale for perfusion and a green-yellow scale for wall motion, a visual indication is provided for which cardiac characteristic is currently displayed. As an example, for a motion change map such as a wall position change map or a wall thickness change map, the spatial displacement of an inner, outer, or medial myocardial wall contour or a change of the wall thickness is computed locally between the various cardiac phases. The local wall displacement or thickness change magnitude is then mapped onto the gray-level scale or the pseudo-color scale, indicating more or less than average variation between the diastole and the systole. As another example, for a perfusion map or a perfusion change map, local hyper- or hypo-perfusion may be computed either on a scan at rest, at stress, or in a late enhancement phase individually, e.g., relative to the median intensity of the entire myocardium, or as change (e.g., a difference or a ratio) between these scans. The perfusion change between different image volumes may be computed using spatial correspondence from either voxel-wise registration, or mesh-based registration, or AHA-segment-based registration. Finally, the contrast attenuation map, or the contrast attenuation change map is then mapped onto the gray-level scale or the pseudo-color scale.

According to an embodiment, a prior three-dimensional smoothing is performed on the change maps. Said smoothing may be, e.g., Gaussian smoothing, or edge-preserving smoothing. That way, the resulting visualization may exhibit finer or coarser granularity, depending on the smoothing filter width.

According to an embodiment, voxelwise change magnitudes are accumulated, averaged and displayed for the standard cardiac segments as defined by the American Heart Association (AHA), to remove noise and for condensed reporting.

In another aspect of the present invention, a computer program element is provided. Said computer program element, when executed by at least one computing unit, is adapted to cause the computing unit to perform the method according to the above description. In particular, the computer program element leads to an improved assessment of the myocardium of the patient and hence to an improvement of the diagnostic certainty. Further embodiments, examples and advantages correspond to those given in the above description.

In yet another aspect of the present invention, an image processing device is provided. Said image processing device is configured to perform the method according to the above description. In particular, the image processing device allows an improved assessment of the myocardium of the patient and hence an improvement of the diagnostic certainty. In particular, the image processing device may be an imaging workstation or a viewer for a picture archiving and communication system (PACS), or a part thereof. The image processing device may comprise a plurality of units to perform the method, e.g., a data input unit, a processing unit, and/or an output unit. Further embodiments, examples and advantages correspond to those given in the above description.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a flowchart of an embodiment of a medical image processing method;
Fig. 2 shows a flowchart of an embodiment of a processing step;
Figs. 3a and 3b show an example of a synoptic representation with a discrete switch;
Figs. 4a to 4c show an example of a synoptic representation with a smooth blend; and
Fig. 5 shows an example of a synoptic representation with interleaved alternating patches.

### DETAILED DESCRIPTION OF EMBODIMENTS

Like numbered elements in these Figures are either equivalent elements or perform the same function. Elements which have been discussed previously will not necessarily be discussed in later Figures if the function is equivalent.

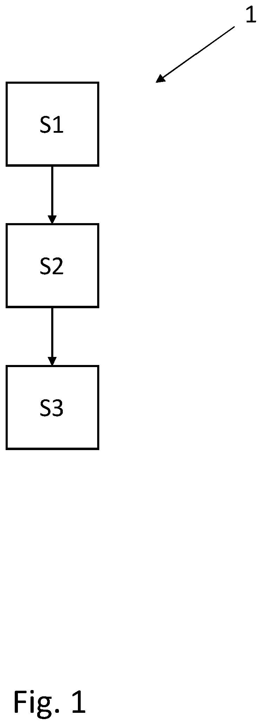

Fig. 1 shows a flowchart of an embodiment of a medical image processing method 1.

The method comprises receiving S1 cardiac image data of a patient relating to at least two different cardiac characteristics. In this context, the patient may be a human, but may also be an animal. The cardiac image data may have been obtained using computed tomography (CT), in particular spectral CT or photon-counting CT. Alternatively, or additionally, the cardiac image data may have been obtained using positron emission tomography (PET) or magnetic resonance imaging (MRI). The cardiac image data relates to at least two different cardiac characteristics. Depending on the characteristics, the cardiac image data may be obtained in a same scan sequence, in different scan sequences, or even in a single scan.

The cardiac characteristics may comprise a perfusion of a myocardium of the patient. In this context, the myocardium may be the entire myocardium of the patient, or a part of the myocardium of the patient, in particular, the part of the myocardium corresponding to the left ventricle of the heart. The perfusion may be determined by the uptake of a contrast agent. In particular, the perfusion may be determined when the patient is at rest or when the patient is at stress, more particularly at induced stress. Further, the perfusion may be measured in an arterial phase, which is also called late systemic-arterial phase or early venous portal phase. Alternatively, the perfusion may be measured in a late enhancement phase, which is also called delayed enhancement phase or delayed phase. Said late enhancement phase is particularly sensitive to tissue with fibrosis. Yet alternatively, the perfusion may be measured in another phase of the contrast enhancement that is deemed useful. Four specific cardiac characteristics for perfusion may be derived from this list: at rest and measured in the arterial phase, at rest and measured in the late enhancement phase, at stress and measured in the arterial phase, and at stress and measured in the late enhancement phase, and other cardiac characteristics can easily be thought of. Additionally, or alternatively, the cardiac characteristics may comprise a change in perfusion of the myocardium of the patient. This may be, in particular, a change between a measurement of the patient at rest and another measurement at stress. Additionally, or alternatively, the cardiac characteristics may comprise a wall thickness of the myocardium. Said wall thickness may be measured, in particular, at a diastole or at a systole, but measurements at other cardiac phases are also possible. Said measurements at specific cardiac phases may be performed by ECG-gated scans of the beating heart. Additionally, or alternatively, the cardiac characteristics may comprise a change in wall thickness of the myocardium of the patient. This may be, in particular, a change between the wall thickness measured at the diastole and the wall thickness measured at the systole. Additionally, or alternatively, the cardiac characteristics may comprise a magnitude of wall motion of the myocardium of the patient. In particular, the magnitude of wall motion may be the magnitude of the motion of the endo-cardiac wall, the magnitude of the motion of the epi-cardiac wall, or the magnitude of the motion of a layer between the endo-cardiac wall and the epi-cardiac wall. The magnitude of wall motion may be determined, e.g., by the motion of the wall between the diastole and the systole. The magnitude of wall motion is understood as a local magnitude and is indicative of myocardial contractility. In summary, there is a plurality of different cardiac characteristics that may be used to generate the synoptic representation. Using at least two cardiac characteristics that are complementary to one another for the synoptic representation results in a particularly advantageous embodiment of the method. Such cardiac characteristics that are complementary to one another are, in particular, perfusion characteristics on the one hand and wall thickness or wall motion characteristics on the other hand.

The method 1 further comprises processing S2 the cardiac image data to generate a synoptic representation of the at least two different cardiac characteristics. In this context, a synoptic representation is a representation that shows the at least two different cardiac characteristics together, such that a combined assessment of the at least two different cardiac characteristics is enabled.

The method 1 also comprises outputting S3 the synoptic representation. Said output may be, e.g., directly to a screen, or via a graphics interface and/or a network to a screen. The output may also be to a memory, such that the synoptic representation can be accessed from the memory for later display and assessment.

The synoptic representation may be viewed by a clinician to assess a myocardium of the patient. In particular, the synoptic representation may be used to assess myocarditis or ischemia, or for cardiac quantification and/or diagnosis. Since at least two different cardiac characteristics are assessed together using the synoptic representation, a spatial correspondence between defect patterns seen in each of the at least two different cardiac characteristics may be identified, leading to an improved assessment of the myocardium of the patient and hence to an improvement of the diagnostic certainty.

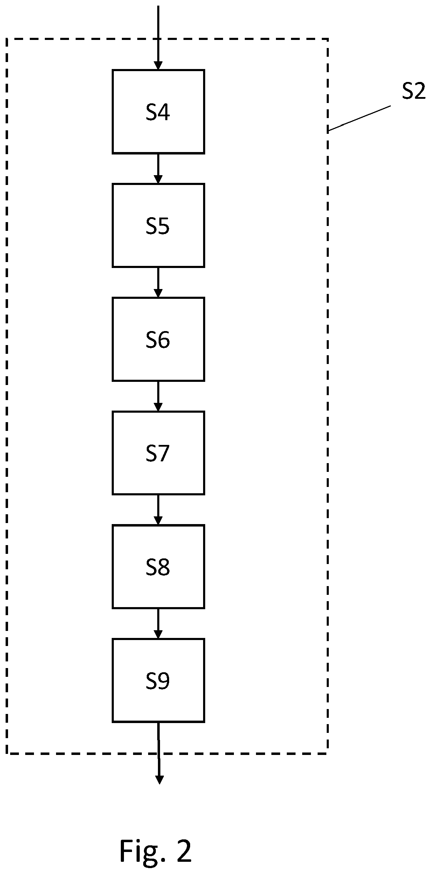

Fig. 2 shows a flowchart of an embodiment of the processing step S2. The processing step S2 includes performing S4 a cardiac segmentation on the volumetric image data to obtain a myocardial region within the volumetric image data, receiving further user input and refining S5 the myocardial region based on the further user input, performing S6 spatial alignment and/or registration, performing S7 a normalization of values of each cardiac characteristic, mapping S8 values of each cardiac characteristic to a gray-level scale or a pseudo-color scale and combining S9 the graphical representations to obtain the synoptic representation. It is to be noted that the processing step S2 does not have to include all of the mentioned steps, in particular, if the received S1 cardiac image data is already pre-processed.

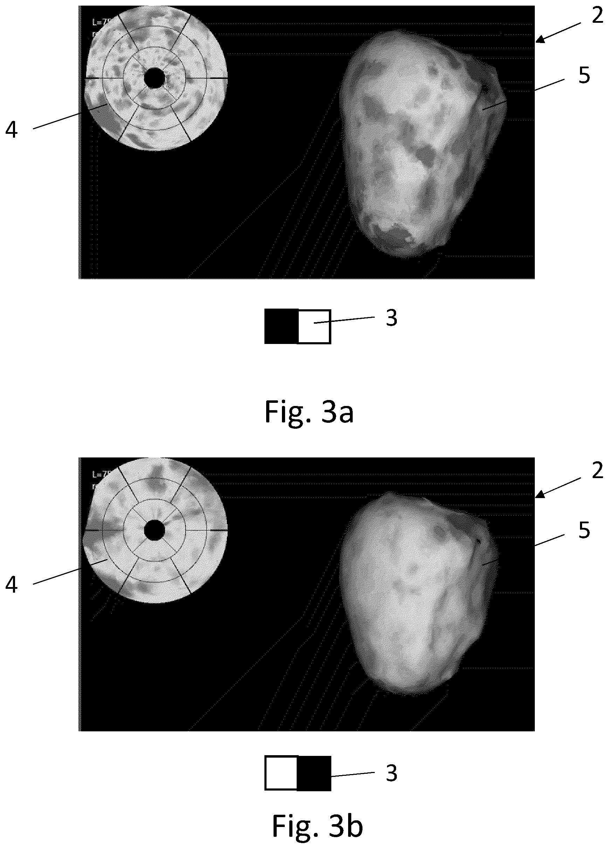

Figs 3a and 3b show an example of a synoptic representation 2 that comprises two graphical representations of the myocardium that temporally changes between two different cardiac characteristics, in this case between a perfusion characteristic shown in Fig. 3a and a wall motion characteristic shown in Fig. 3b. In this example, the temporal change is a discrete switch, i.e., either the one or the other cardiac characteristic is shown. The temporal change may be controlled by a user using a switch 3, wherein one position of the switch corresponds to one cardiac characteristic and the other position of the switch corresponds to the other cardiac characteristic. Of course, instead of using user input, the temporal change may also be a period change. The graphical representations include a 17-segment model view 4 and a three-dimensional surface rendering decorated with a texture 5. However, other graphical representations, such as a two-dimensional projection and/or a color overlay on a cross-sectional view may also be included in the synoptic representation.

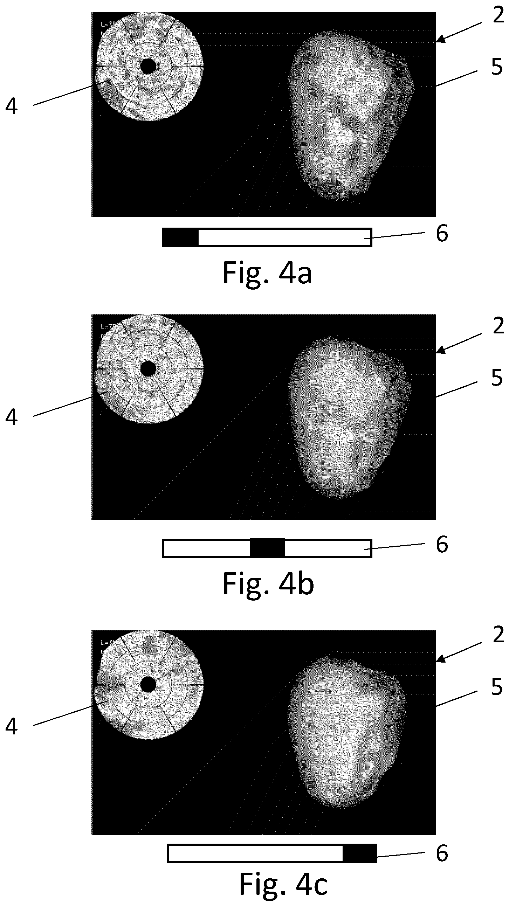

Figs 4a to 4c show another example of a synoptic representation 2. Instead of the switch 3 of the example shown in Figs 3a and 3b, a slider 6 is provided that allows the user to control a smooth blend between the two cardiac characteristics. In Fig. 4a, one cardiac characteristic is selected, in Fig. 4c, the other cardiac characteristic is selected, and in Fig. 4b, an intermediate state between the two cardiac characteristics is selected, wherein said intermediate state may have been obtain by interpolation, e.g., by linear interpolation.

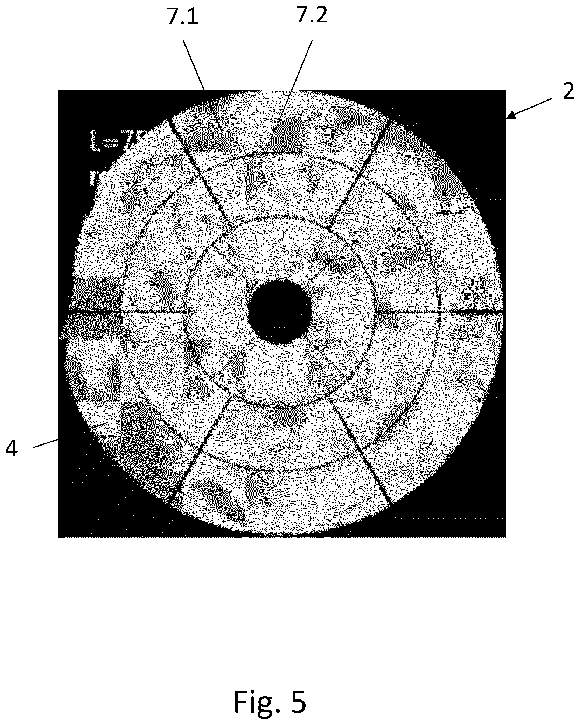

Fig. 5 shows yet another example of a synoptic representation 2. In this synoptic representation, the graphical representation, which is here the 17-segment model view 4, has interleaved alternating patches 7.1 and 7.2, wherein only two of said patches have been provided with reference numerals. Said patches 7.1 and 7.2 show alternatingly one cardiac characteristic and another cardiac characteristic, such that both cardiac characteristics can be seen at the same time.

Further embodiments of the present invention which are not shown in the Figures relate to a computer program element, which, when executed by at least one computing unit, is adapted to cause the computing unit to perform the medical image processing method (1) and to an image processing device, configured to perform the medical image processing method (1).

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 1: medical image processing method
- 2: synoptic representation
- 3: switch
- 4: 17-segment model view
- 5: three-dimensional surface rendering
- 6: slider
- 7.1: patch
- 7.2: patch

- S1: receiving cardiac image data
- S2: processing the cardiac image data
- S3: outputting the synoptic representation
- S4: performing a cardiac segmentation
- S5: refining the myocardial region
- S6: performing spatial alignment and/or registration
- S7: performing a normalization
- S8: mapping values to scales
- S9: combining the graphical representations

## Claims

1. A medical image processing method (1), comprising:
receiving (S 1) cardiac image data of a patient relating to at least two different cardiac characteristics;
processing (S2) the cardiac image data to generate a synoptic representation (2) of the at least two different cardiac characteristics; and
outputting (S3) the synoptic representation (2).

2. The method (1) according to claim 1, wherein the cardiac characteristics comprise:
a perfusion of a myocardium of the patient, wherein, in particular
the patient is at rest; or
the patient is at stress; and/or
the perfusion is measured in an arterial phase; or
the perfusion is measured in a late enhancement phase;
a change in perfusion of the myocardium of the patient;
a wall thickness of the myocardium of the patient, wherein, in particular
the wall thickness is measured at a diastole; or
the wall thickness is measured at a systole;
a change in wall thickness of the myocardium of the patient; and/or
a magnitude of wall motion of the myocardium of the patient.

3. The method (1) according to claim 1 or 2, wherein the synoptic representation (2) comprises at least one graphical representation of the myocardium that temporally changes between the at least two different cardiac characteristics.

4. The method (1) according to claim 3, wherein the temporal change between the at least two different cardiac characteristics is a discrete switch and/or a smooth blend.

5. The method (1) according to claim 3 or 4, wherein the step of processing (S2) the cardiac image data comprises receiving user input, and the temporal change between the at least two different cardiac characteristics is controlled by the user input.

6. The method (1) according to any one of claims 1 to 5, wherein the synoptic representation (2) comprises at least one graphical representation of the myocardium with interleaved alternating patches (7.1, 7.2) corresponding to the at least two different cardiac characteristics, in particular resembling a checkerboard pattern.

7. The method (1) according to any one of claims 1 to 6, wherein the at least one graphical representation comprises at least one out of:
a 17-segment model view (4);
a two-dimensional projection;
a color overlay on a cross-sectional view; and
a three-dimensional surface rendering decorated with a texture (5).

8. The method (1) according to any one of claims 1 to 7, wherein the cardiac image data is volumetric image data and the step of processing (S2) the cardiac image data comprises performing (S4) a cardiac segmentation on the volumetric image data to obtain a myocardial region within the volumetric image data.

9. The method (1) according to claim 8, wherein the step of processing (S2) the cardiac image data comprises receiving further user input, and the myocardial region is refined (S5) based on the further user input.

10. The method (1) according to any one of claims 1 to 9, wherein the step of processing (S2) the cardiac image data comprises performing (S6) spatial alignment and/or registration.

11. The method (1) according to any one of claims 1 to 10, wherein the step of processing (S2) the cardiac image data comprises performing (S7) a normalization of values of each cardiac characteristic.

12. The method (1) according to any one of claims 1 to 11, wherein the step of processing (S2) the cardiac image data comprises mapping (S8) values of each cardiac characteristic to a gray-level scale or a pseudo-color scale.

13. A computer program element, which, when executed by at least one computing unit, is adapted to cause the computing unit to perform the method (1) according to any one of claims 1 to 12.

14. An image processing device, configured to perform the method (1) according to any one of claims 1 to 12.
